Europäisches Patentamt

European Patent Office

Office européen des brevets

(19)

(11) Publication number: **0 203 238**
**A1**

# EUROPEAN PATENT APPLICATION

(12)

(21) Application number: 85307785.7

(22) Date of filing: 28.10.85

(51) Int. Cl.⁴: **G 01 N 33/52**, G 01 N 33/543, G 01 N 33/94 // G01N33/577

(30) Priority: 24.01.85 US 694506
22.02.85 US 704157

(43) Date of publication of application: 03.12.86
Bulletin 86/49

(84) Designated Contracting States: **AT BE CH DE FR GB IT LI LU NL SE**

(71) Applicant: **QUIDEL, 11077 North Torrey Pines Road, La Jolla, CA 92037 (US)**

(72) Inventor: **Lee, Theodore Tsan-Tsung, 4965 Via Papel, San Diego California (US)**
Inventor: **Katz, David H., 1775 La Jolla Road, La Jolla California (US)**

(74) Representative: **Sommerville, John Henry et al, SOMMERVILLE & RUSHTON 11 Holywell Hill, St. Albans Hertfordshire, AL1 1EZ (GB)**

(54) **Apparatus for use in detecting ligands in solution.**

(57) An assay system and apparatus for detecting ligands in solution, typically, drugs of abuse such as morphine in body fluids e.g., urine, and semi-quantitatively determining the same by comparison with a reference.

- 1 -

## ASSAY FOR DRUGS OF ABUSE

### FIELD OF THE INVENTION

This invention relates to the detection and semi-quantitative determination of drugs of abuse. The particular drug, e.g., morphine, cocaine, amphetamine, etc., or a metabolite thereof, is detected by contacting the body fluid, e.g., urine, with an immunologically-specific dipstick which has an indicator pad and a reference pad.

### BACKGROUND OF THE INVENTION

Many procedures, techniques and reagents have been reported for the detection of particular ligands in body fluids. Among the important application of the principles involved in the detection of ligands is the detection of drugs of abuse for both medical and legal reasons. Ligands which have been detected using what may be broadly described as immunochemical techniques include various drugs which have a variety of physiological effects. Such drugs include narcotics, hypnotics, sedatives, analgesics, antipyretics, anesthetics, psychotogenic drugs, muscle relaxants, nervous system stimulants, anticholinesterase agents, parasympathomimetic agents, sympathomimetic agents, neuromuscular agents, histamines, antihistamines, cardiovascular drugs, antiarrhythmic drugs, antihypertensive agents, vasodilator drugs, diuretics, antibiotics, hormones, vitamins, tumor cells, bacterial and viral proteins, toxins, blood proteins and their metabolites.

Included in the drugs which are of particular interest are alkaloids, steroids, polypeptides and proteins.

Ligands may be quite small, e.g., low molecular weight organic compounds, or very large, having molecular weight as high as 100,000 or more. Ligands of interest in the present assay include antigens and, very importantly, haptens. Haptens are substances which, upon being injected as simple chemicals, do not give rise to antibodies. However, antibodies can be raised against haptens when they are conjugated to antigenic carriers prior to using them for immunization. Some ligands of interest in this invention are described and discussed in some detail in U.S. Patent No. 4,067,774, Rubenstein and Ullman, January 10, 1978. U.S. Patent No. 4,067,774 also includes a specific and extended description of the drugs of abuse, referred to therein as illicit drugs, which are of a special interest in the present invention. These drugs include opiates such as morphine and heroin, meperidine and methadone. Other drugs of interest include the amphetamines, narceine, epinephrine, ephedrine and L-Dopa. The description of these drugs of abuse and their chemical characteristics found in U.S. Patent No. 4,067,774, is incorporated herein and reference is made to the aforesaid patent for a more comprehensive discussion of the various ligands which may be determined according to the principles of this invention.

Rubenstein and Ullman describe in the aforesaid U.S. Patent No. 4,067,774 the formation of enzyme-bound-ligands of particular composition.

U.S. Patent No. 3,817,837 describes a method of carrying out a homogeneous enzyme immunoassay which may be applied to the detection of drugs of abuse.

U.S. Patent No. 4,069,105 describes other enzyme conjugates with various ligands, lidocaine in particular. Cocaine derivatives are conjugated with various enzymes and with other compounds to provide reagents for immunoassay techniques according to techniques described by Soffer and Schneider, U.S. Patent No. 4,123,431, October 31, 1978.

Rubenstein and Ullman, U.S. Patent No. 4,190,496, have described a method for determining the ligands and receptors of interest in this invention using the principle of reduction in enzymatic activity of an enzyme-bound-ligand.

Particular malate dehydrogenase conjugates for enzyme immunoassays are described by Ullman and Rubenstein, U.S. Patent No. 4,191,613.

- 4 -

Other systems and compositions which are suitable for use in the detection of drugs of abuse and other ligands of interest in the present patent are described in U.S. Patent No. 4,193,983, March 18, 1980, Ullman and Brinkley; Leute and Bolz, U.S. Patent No. 4,197,237, April 8, 1980; Ullman and Schwarzberg, U.S. Patent No. 4,199,559, April 22, 1980; Rubenstein and Ullman, U.S. Patent No. 4,203,802, May 20, 1980; Zuk and Maggio, U.S. Patent No. 4,208,479, June 17, 1980; U.S. Patent No. 4,233,402, November 11, 1980, Maggio, Wife and Ullman. In this latter patent, a method of enzyme channeling is described. Other disclosures of interest include Singh and Pirio, U.S. Patent No. 4,235,969, November 25, 1980; Litman and Ullman, U.S. Patent No. 4,299,916, November 10, 1981; U.S. Patent No. 4,328,311, May 4, 1982, Wroley and Leung; Rubenstein and Ullman, U.S. Patent No. 4,376,825, March 15, 1983; Rubenstein and Ullman, U.S. Patent No. 4,423,143; and, Zuk and Litman, U.S. Patent No. 4,435,504.

Of general interest, as related to enzyme-linked immunoassay methods, is U.S. Patent No. 3,654,090, Schuurs and VanWeemen, April 4, 1972.

There are a great many kits and test strips which involve the enzyme-linked immunoassay principle. The most pertinent disclosure, as related to the present invention, which is known to the inventors is that of Litman, et al., "An Internally Referenced Test Strip

- 5 -

Immunoassay for Morphine", Clinical Chemistry, Volume 29, No. 9, Pages 1598-1603 (1983). The Litman, et al. internally referenced test strip is described as being composed of two active surfaces, each of which contains co-immobilized glucose oxidase and antibody. The indicator pad contains antibody directed against the drug, and the color that develops on its surface is inhibited by the presence of the drug in the sample. The reference pad contains anti-peroxidase and is used to set the assay detection limit, and normalize for variants in temperature, timing, and sample interference. By adjusting the anti-peroxidase loading, the color that is produced on the reference pad can be made equal to that produced on the indicator pad when the analyte concentration is equal to the detection limit of the assay. This test strip is described with particular applicability to the detection of morphine.

While the Litman, et al. internally referenced test strip has some apparent advantages, it would suffer from a significant disadvantage in that it would be extremely difficult, nearly impossible in production environments, to provide consistent anti-peroxidase loading on the reference pad. This requires very tight quality control and inevitably would result in a great deal of rejected product.

The present invention also provides an internally referenced pad, but overcomes the difficulties presented in the Litman, et al. internally referenced test strip. The present invention utilizes the enzyme-linked immunoassay techniques which are generally described in the cited prior art and a large volume of published literature and patents, and is distinguished from the prior art in that the problem of controlling the loading of the reference pad has been overcome by the apparatus, methods and techniques described hereinafter.

## SUMMARY OF THE INVENTION

The present invention is directed to apparatus for use in determining the presence of a ligand. The apparatus comprises a reference solid support area comprising a first component of a reference coupling pair which is capable of coupling with a second component of a reference coupling pair. The second component of the reference coupling pair is labeled with a moiety which can

///

///

///

///

///

///

///

///

///

///

///

be detected and measured. This second component is referred to as the reference conjugate. A test solid support area is also included in the apparatus, the test area comprising a receptor of an immunochemically binding pair. The other component of the immunochemically binding pair comprises the ligand to be determined, i.e., the test ligand. The receptor is capable of immunochemically binding the test ligand and an immunochemically identical ligand labeled with same moiety used to label the reference conjugate. In a preferred form, the reference coupling pair comprises avidin and biotin, and the immunochemically binding pair comprises a pair selected from the group of pairs consisting of (a) an antigen and an antibody to said antigen, (b) a hapten and an antibody to said hapten, and (c) a protein and an antibody to the protein.

The process of this invention comprises the steps of inserting a test apparatus, which comprises the reference and test solid support areas described above, into a test solution suspected of containing the test ligand, e.g., morphine. Reference conjugate and ligand conjugate of the immunochemically binding pair in known quantities are added to the test solution. The test solution and the apparatus are incubated or otherwise allowed to react for a sufficient period of time to effect immunochemical binding reactions and chemical binding reactions between, respectively, the immunochemically coupling pair and the reference coupling pair. The
reference area, after the above reaction, contains
reference conjugate from the test solution. The test area
contains test ligand and ligand conjugate. The test
ligands and labelled ligands on the test area are
proportional to the amounts of test ligand and ligand
conjugate in the test solution. The test apparatus is
removed, washed if necessary, and the labeling moiety is
developed, if necessary, and detected. Any signal
developed, e.g., a colored entity, must remain where it is
generated and not dissolve or diffuse away (i.e., there
must be some affinity between the colored entity and the
solid support.) In the preferred form of the invention,
the label is an enzyme which, upon being developed,
produces a distinctive color which remains where it is
developed. Radiolabels or fluorescent labels, on moieties
which are stable where produced, may, however, be used.
In the preferred form in which an enzyme label is used,
the color on the test area is compared with the
color on the reference area to give a semi-quantitative
measure of the amount of test ligand present in the test
solution.

In a particularly preferred form, the invention
comprises a test strip or stick which has thereon two
areas, referred to for convenience as pads, a reference
pad, and a test pad. The reference pad consists
essentially of one component of a reference coupling pair, such as avidin. The test pad consists essentially of an immobilized receptor for the test ligand.

In another preferred form, the test stick comprises a single reference pad and indicator pads for detecting two different test ligands.

In a preferred form of the process of the invention, the test apparatus in the form of a strip or stick is allowed to react with a solution suspected of containing the test ligand, to which is added known concentrations of the reference conjugate and of the ligand of interest to which the same enzyme used in the reference conjugate has been conjugated, i.e., the ligand conjugate. The known amount of ligand conjugate competes for sites on the test pad with the test ligand, while the reference conjugate independently reacts with first component of the reference couple on the reference pad without interference by the test ligand. To the extent reaction times, temperatures, concentrations, interfering constituents and other variables affect the assay, the effect is substantially the same for both the ligand conjugate and the reference conjugate. The enzyme is developed to produce the characteristic color. The color on the reference pad is the standard and is a function of the available reference conjugate. The color of the test pad is a function of the portion of the available sites on the test pad occupied by ligand conjugate and inversely proportional to the amount of test ligand in the sample.

- 10 -

The present invention obviates one of the major difficulties faced in earlier systems using a reference pad. In earlier assays, it was necessary to control with great precision the amount of antibody, or other immunochemical receptor, on the reference pad. In production, this is an almost impossible, and certainly impractical, procedure which would certainly result in a high proportion of rejected product. According to the present invention, it is not necessary to maintain such high precision in the manufacture of the test stick or strip. Control is imposed in the amount of reference conjugate, and ligand conjugate, added to the test solution. It is a very easy matter to "fine tune" the addition of these materials to the test solution. Quantities are easily controlled and great precision is readily attainable.

The invention, in one of its more preferred forms, is a kit suitable for use in testing for the presence of test ligand in body fluids, e.g., testing for morphine in urine. The kit comprises test apparatus as described above, along with ligand conjugate and reference conjugate at predetermined concentrations. The kit may also include vessels and other apparatus for carrying out the test. It is within the scope of this invention to include more than one reference pad and/or more than one test pad in the same apparatus.

## DESCRIPTION OF THE DRAWINGS

Figure 1 is a graph showing the response of test

- 11 -

and reference pads to different concentrations of morphine using ALP-morphine as the ligand conjugate, ALP-biotin as the reference conjugate and avidin on the reference pad.

Figure 2 is a graph showing the response of test reference pads to different concentrations of morphine using ALP-DNP as the reference conjugate and immobilized murine monoclonal anti-DNP on the reference pad.

Figure 3 is a graph showing the response of test and reference pads to different concentrations of Vitamin H (biotin) using ALP-DNP as the reference conjugate, and immobilized murine monoclonal anti-DNP on the reference pad.

Figures 4 and 5 are, respectively, front and side views of a simple dipstick construction, including a reference pad or area, and an indicator pad or area.

Figure 6 is an alternative form of the invention, having one reference and two test pads.

## DESCRIPTION OF THE INVENTION

### DEFINITIONS

The following terms used herein are defined at this point for convenience:

**Ligand.** The term "ligand" is used here in the immunochemical sense and includes the components of any pair which binds together through or as the result of immunochemically determinant structures. Ligands include hapten-antibody and antigen-antibody pairs, and both polyclonal and monoclonal antibodies. The term

- 12 -

"antigen" in this definition is used broadly to include any substance which induces an antibody reaction in animals.

Receptor. A receptor, as used here, means a substance which will immunochemically bind a ligand, with particular reference to the ligand to be determined, and typically will be the antibody to the hapten or the antigen.

Reference coupling pair. Reference coupling pair (or reference couple) refers to a pair of compounds which have specific affinity for each other. The affinity may be chemical or immunochemical. Typical of coupling pairs are: Avidin and biotin; hapten and antibody to the hapten; polyanion and cation; or, polycation and anion.

Reference conjugate. Reference conjugates are formed by conjugating one member of the reference couple with a labeling moiety, e.g., a labeling enzyme.

Ligand conjugate. Ligand conjugates are formed by conjugating ligands of the immunochemical type to be determined with a labeling moiety.

LIGANDS

Ligands of interest with respect to this invention include any ligand which may be found in body fluids or tissue which is soluble in or can be solubilized in a test solution.

Among the ligands of particular interest are the "drugs of abuse." Drugs of abuse include a wide spectrum of drugs having a variety of physiological reactions when ingested or injected. Among the most common of these drugs of abuse are drugs which act as narcotics, hypnotics, sedatives, psychotogenic drugs, muscle relaxants, and nervous system stimulants. Particularly important drugs of abuse to which this invention is applicable, include opiates such as morphine, cocaine, amphetamines, barbiturates, phencyclidine, tetra-hydrocannibinol (THC), methadone, Valium (oxazepam), and their metabolites.

Opiates which can be detected according to the principle of this invention include morphine, heroin, hydromorphone, oxymorphone, metopon, codeine, hydrocodone, dihydrocodeine, dihydrohydroxycodeinone, pholocodine, dextromethorphan, phenaxocine and dionin.

Catacholamines, and the related compounds epinephrine, amphetamines, and other related compounds are also subject to determination by the apparatus and methods of this invention.

Barbiturates such as veronal, medinal, luminal, prominal, soneryl, nembutal, amytal, dial, phenadorn, seconal, evipan, phenobarbital and pentothal may be detected according to the principles of the present invention.

Cocaine, a drug now being very widely abused, and its analogs, and marijuana, also widely abused, may be detected by the present invention.

- 14 -

Tranquilizers such as meprobamate, and the benzdiazocycloheptanes are also abused and are subject to detection according to this invention.

Amino acids, peptides and polypeptides are also subject to detection according to this invention, as are other antigenic substances and organisms to which a specific monoclonal antibody can be produced.

Other substances which are subject to detection by the present invention include antibiotics such as penicillin, chloromycetin, actinomyucetin, tetracycline, terramycin, nucleic acids or derivatives, such as nucleosides and nucleotides, steroids, hormones, insecticides, fungicides, bacteriocides and nematocides.

## ENZYMES

A wide variety of labeling enzymes that can be used in homogeneous type EIA (e.g. Syva's Emit System) have been reported in the literature. Typical of such enzymes are the oxidoreductases and hydrolases. The criteria for selecting those enzymes is well known, and has been discussed at some length by Rubenstein and Ullman, U.S. Patent No. 4,067,774. The criteria for selecting an enzyme for use in the present invention are quite different, however, than the criteria discussed by Rubenstein and Ullman. First, the enzyme-ligand conjugate should not be grossly inhibited upon binding by the antibody. This is also true of the reference conjugate. Second, the colored product generated by the enzyme must bind to the solid support used in the present invention. Particular enzymes suitable for use as labels in the present invention include: horseradish peroxidase (HRP), beta-galactasidase, acid phosphatase and alkaline phosphatase (ALP), which is used as exemplary of the many available enzymes.

## OTHER LABELS

Radioactive labels, a number of which are well known and generally used in the art, fluorescent labels and catalytic labels may be used. However, the preferred class of labels are the enzymes, as discussed, because of the simple and safe manner in which they can be employed without the need of specialized instrumentation or particular safety precautions.

## CIRCUMSTANCES LEADING TO THE INVENTION

In enzyme immunoassays that utilize solid substrata, be it sandwich type or competitive-type enzyme immunoassay, it is usually possible to find enzyme substrates that will form colored insoluble products that bind strongly to the solid matrix. For example, 5-Bromo-4-chloro-3-indolyl phosphate, a substrate for alkaline phosphatase (ALP), forms a blue indigoid dye, which binds strongly (or deposits itself, if you wish) to paper. Paper (cellulose) and other hydrogen-bond forming solid matrices (e.g. nylon) are usually good choices for this purpose. This property can be utilized to conveniently terminate an assay by just removing the solid matrix from the substrate solution and subsequently blotting it to dryness, resulting in quenched enzyme activity and a display of the end-point color. Furthermore, it is now possible to do "internal" referencing. One can place in the sample solution, together with the first solid matrix, a second one on which is immobilized an appropriate amount of a suitable substance, and be able to determine the relative concentration of the analyte in question by comparing the color displayed on the first solid matrix to that on the second one, which will always correspond to a certain pre-set analyte concentration, regardless of what the samples may be. Thus, there is no need to run standards along with samples (external referencing). In fact, internal referencing should be superior to external referencing in most cases. This is because: (i) sample matrices (especially urine samples) vary to a great extent, which may affect both enzyme activity and the nonspecific stickiness of the enzyme conjugates to the solid matrix; (ii) variations in sample incubation time, washing technique and substrate incubation time.

An example of internal referencing, relating to a competitive type enzyme immunoassay is a dipstick assay for morphine described by Litman, et al., Clinical Chemistry, 29, 1598-1603 (1983). This dipstick also has two pads: The indicator pad contains immobilized antibody against morphine, whereas the reference pad contains

- 17 -

antibody against horseradish peroxidase (HRP). The
dipstick is first placed in a urine sample and then in a
developer solution containing HRP-morphine conjugate. The
system is so designed that the reference pad will display
color corresponding to about 300 ng/ml morphine, and,
therefore, a less intense blue color displayed on the
indicator pad indicates the presence of more than 300
ng/ml of morphine.

The idea of using anti-HRP on the reference pad
in the above example is fine, except that it is usually a
very painstaking task to control (or fine-tune) the amount
of antibody immobilized on the reference pad so that it
will display color corresponding to a certain pre-set
analyte concentration. This is especially true in
manufacturing a product like this. In the first place,
one can never be sure that a large scale production run
will yield precisely the same product as in a small scale
trial run. If it turns out that the reference pad does
not match the indicator pad, the whole lot of reagent
paper for the reference pad has to be either discarded or
put aside for a second round of matching with a different
lot of indicator pad. Secondly, as a corollary of the
above, it can never be guaranteed that the same lot of
reference pad will match more than one lot of indicator
pad and enzyme conjugate. This necessitates small trial
runs before each production run.

According to this invention, a system to confer

- 18 -

both elegant precision in fine-tuning the color on the reference pad to correspond to the desired analyte concentration and great ease and flexibility in the manufacturing process has been devised.

## DETAILED DESCRIPTION AND SPECIFIC EXAMPLES

The invention involves mainly the concept of "reference conjugates." A "reference conjugate" is, in the preferred form, an enzyme that has been chemically modified to contain one or more covalently bonded groups of identical chemical structure which does not exist on the original enzyme molecule and bears no resemblance to the substance being analyzed, and will be bound by a binder substance immobilized on a solid substratum, the reference area or pads, that specifically recognizes and binds the group. By simply adjusting the concentration of this reference conjugate, one can fine-tune the color on the reference area or pad very precisely and with great ease to that corresponding to the desired level of analyte concentration. This reference is totally independent of the analyte in sample because of the complete lack of affinity of the binder substance for the analyte, and, therefore, can be used as an internal reference. A table of some representative groups and corresponding binder substances that can be used in this system is shown below.

- 19 -

TABLE I

| Binding Group in Reference Conjugate | Binder Substance on Solid Substratum |
|---|---|
| biotinyl | avidin |
| hapten | monoclonal anti-hapten |
| polyanion (polycation) | cation (anion) |

Compounds belonging to the hapten category are vast and potentially most useful. One can almost select any structure that one desires and raise monoclonal antibodies against it.

The following are three examples pertaining to the use of reference enzyme.

EXAMPLE A

I. Reagents:

(i) Enzyme conjugate cocktail: contains 40 nM ALP-morphine conjugate and 7 nM ALP-biotin conjugate (the reference conjugate) in conjugate buffer.

(ii) Dipstick: the reference pad (3/16" x 1/8") is attached to one end of a plastic handle (3/16" x 2 1/2") and the indicator pad (3/16" x 1/8") is placed next to it. The reference pad contains avidin immobilized on paper. The indicator pad contains rabbit anti-morphine immobilized on paper.

(iii) Developer Solution: 5mM

- 20 -

5-Bromo-4-chloro-3-indolyl phosphate in
0.3M AMP buffer at pH 10.15 with 0.02%
NaN$_3$.

II.  Assay Procedure:

   (i)    Pipette 400 %l of sample into a 10 x
         50 mm glass tube.

  (ii)    Add 100 %l of enzyme conjugate
         cocktail.  Vortex.

 (iii)    Place dipstick in solution and
         incubate for 10 minutes at room
         temperature.

  (iv)    Remove dipstick.  Wash for 20 seconds
         under tap water.

   (v)    Place dipstick in developer solution
         and incubate for 10 minutes at room
         temperature.

  (vi)    Remove dipstick and blot on a clean
         paper towel.

 (vii)    Blue color is quantitated by reading
         the pads on a MacBeth reflectance
         photometer.

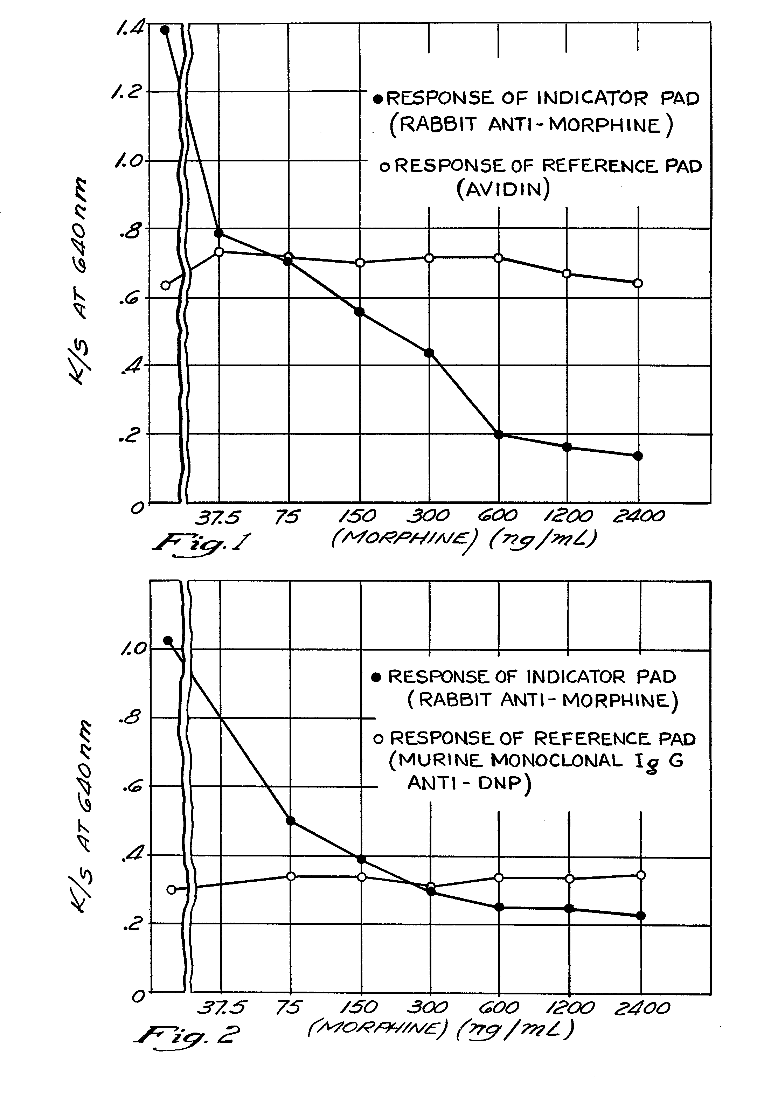

Figure 1 shows the responses of both the
indicator and the reference pad to samples containing
different concentrations of morphine.  The quantity K/S
plotted on the Y-axis is related to reflectance R by the
equation K/S = $(1-R)^2/2R$, and is linearly proportional to
the amount of the blue dye deposited on the pads.  One can see that the amount of the blue dye on the indicator pad decreases with increasing morphine concentration, whereas that on the reference pad remains constant at a level corresponding to 75 ng/ml morphine. Therefore, without even running morphine standards, one can determine if the morphine concentration in the sample is greater or less than 75 ng/ml by just comparing the colors on the two pads.

## EXAMPLE B

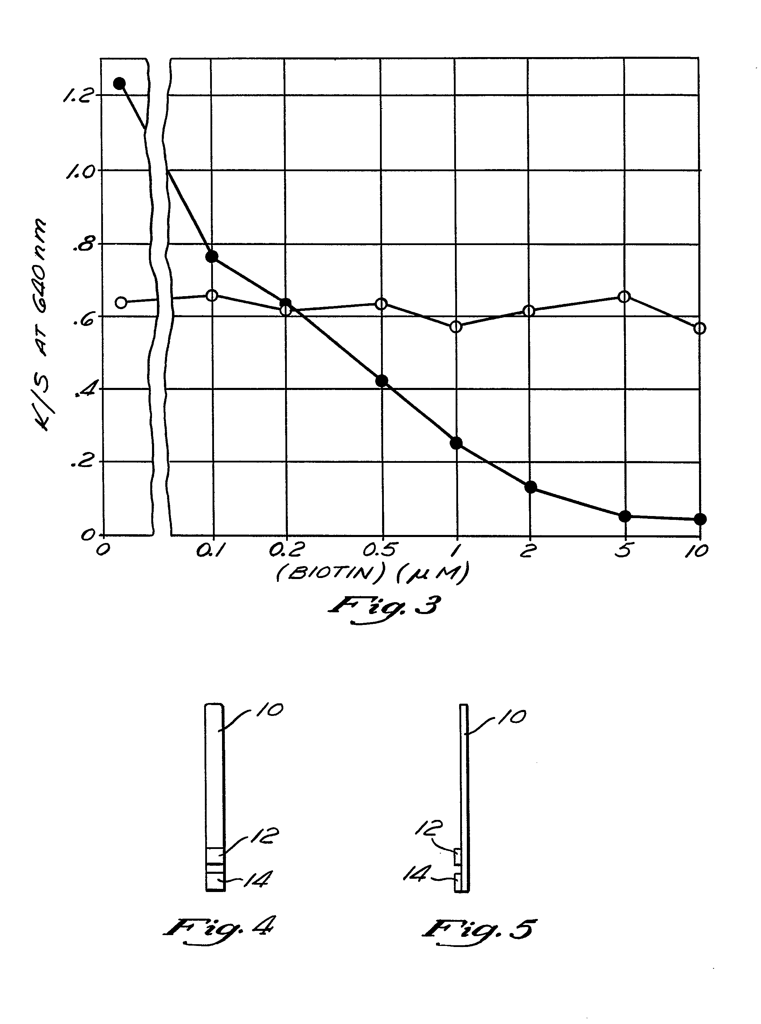

This is similar to Example A, except that the reference enzyme is an alkaline phosphatase-2,4-dinitrophenyl conjugate and the reference pad contains an immobilized murine monoclonal anti-DNP. Figure 2 shows that similar results are obtained as in Example A.

## EXAMPLE C

In Examples A and B, the invention has been used in competitive-type solid phase enzyme immunoassays. This example illustrates the use of the invention in a sandwich-type ELISA to determine hCG concentration in a sample. The enzyme conjugate cocktail consists of ALP-anti-hCG conjugate and ALP-biotin conjugate (the reference conjugate). The reference pad contains immobilized avidin. The concentration of ALP-biotin conjugate may be adjusted so that the color on the reference pad corresponds to an hCG concentration of, for example, 10 mIU/ml. A more intense blue color on the indicator pad indicates the presence of more than 10 mIU/ml of hCG in the sample. This eliminates false positives from the assay.

### EXAMPLE D

The ligand to be detected can be quantitatively determined above or below the concentration level of the reference coupling pair. This is illustrated by the determination of Vitamin H (biotin) according to the following example. Four hundred microliters of sample containing Vitamin H at various concentrations was pippetted into a 10 x 50 mm glass tube. One hundred microliters of enzyme conjugate, containing 23 picomoles of ALP-biotin and of ALP-DNP in conjugate buffer, was added to the sample. A dipstick was inserted in the resulting solution and incubated for 15 minutes. The dipstick was of the type illustrated in Figures 4 and 5, comprising a plastic handle 3/16 inch by 2 1/2 inch with a reference pad and an indicator pad 3/16 x 1/8 inch containing, respectively, murine monoclonal antibody against DNP and avidin immobilized on the paper pad. Following incubation at room temperature, the dipstick was removed and washed 20 seconds under tap water and placed in a solution of 5mM 5-bromo-4-chloro-3-indoyl phosphate in 0.3 M AMP buffer at pH 10.15 with 0.02 percent $NaN_3$. After 15 minutes in the developer at room temperature, the dipstick was removed and blotted on a paper towel. The blue color of the two pads was read on a MacBeth reflectance photometer.

- 23 -

As shown in Figure 3, the quantity K/S plotted on the Y-axis is related to the reflectance R by the equation $K/S = (1-R)^2/2R$, and is linearly proportional to the amount of blue dye deposited on the pads. The amount of blue dye on the indicator pad decreases with increasing concentration of biotin in the sample, whereas the color on the reference pad remains constant at a level corresponding to 0.2%M biotin. Thus, even without running biotin standards, one can determine if the biotin concentration in the sample is greater than or less than the 0.2%M reference level simply by comparing the colors on the two pads.

As depicted in Figures 4 and 5, the apparatus of this invention may, preferably, but not necessarily, take the form of a dipstick having a support, indicated by numeral 10, of wood, paper, plastic, or other semi-rigid material, a reference solid substratum pad, numeral 14, treated, respectively, as described hereinbefore.

A further refinement is fully within the scope of this invention, namely, the use of more than one reference and/or more than one indicator pad per stick. One clear use of the invention is to have two or more reference pads at differing reference levels. Thus, using the illustrative example of Example 3 just discussed, three reference pads corresponding to 0.2%M, 0.4%M and 0.6%M may be incorporated in a single stick, thus giving a very broad range of quantitative or semi-quantitative

- 24 -

determination.

## EXAMPLE E

In this example, the dipstick has three pads as shown in Figure 6. The reference pad 22 on stick 20 is the reference for two pads 24 and 26 for antibarbiturate and antimorphine respectively.

Test pad 24 is on the end of the strip, and contains immobilized rabbit anti-barbiturate. The reference pad 22 is next to test pad 24, and contains immobilized rabbit anti-FITC. Anti-FITC is anti-fluorescein isothiocyanate. Test pad 26 contains immobilized rabbit anti-morphine.

To 400 µl sample was added 100 µl enzyme conjugate cocktail containing ALP-barbiturate, ALP-morphine, and ALP-FITC. A strip was then placed in the solution and incubated for 10 minutes, after which it was rinsed under tap water for 25 seconds and developed for 10 minutes. The results are shown in Table II.

### TABLE II

#### Blue Color Intensities on Pads

| Samples | Test Pad 24 | Reference Pad 22 | Test Pad 26 |
|---|---|---|---|
| Buffer | ++++ | +++ | ++++ |
| Buffer + Morphine | ++++ | +++ | + |
| Buffer + Barbiturate | + | +++ | ++++ |
| Buffer + Morphine + Barbiturate | + | +++ | + |

### DISCUSSION

It should be noted that the choice of the group

- 25 -

to be linked to the reference enzyme is important.
Ideally, it should not resemble anything that could
possibly be present in the sample.

Because of the special design of the reference
enzyme, one can vary its concentration independently
without perturbing the interaction between the main enzyme
conjugate and the indicator substratum.  The amount of
binder substance immobilized on the reference substratum
is therefore not critical at all because of this infinite
flexibility in varying the concentration of the reference
enzyme.  One would try to maximize the amount of
immobilized binder substance on the reference substratum,
and the fine-tuning of the color on which could then be
accomplished with ease and precision by varying the
concentration of the reference enzyme.  The problems
encountered in prior manufacturing practices no longer
exist.  One can match any lots of indicator pad, reference
pad, reference enzyme and main enzyme conjugate by simply
varying the concentration of the reference enzyme, which
is also tremendously simpler and faster than doing
small-scale runs trying to immobilze different amounts of
antibody on paper, a process which takes a lot of time and
effort.

There are many ways in which the present
invention may be used with different reagents.  For
example, the dye does not have to be colored.  It can be
UV-absorbing, thus requiring a reflectance photometer, or fluorescent. The solid substratum, i.e., the test or reference area or pad, is not limited to paper. Anything which binds the appropriate dye will do.

Further, more than one reference substratum may be used in an assay system. In this case, more than one reference enzyme and binder substance will be needed. Using two reference enzymes and their corresponding binder substances, for example, two reference values may be set for an analyte. Thus, for example, low and high normal values of the analyte, or the first and second phases of a disease, may be detected. For more than one reference substratum, it is possible to immobilize more than one binder substance on the same substratum, which would bind all the relevant enzymes. This approach can be applied to the two-reference system, where one reference substratum contains binder substance A, and the other one contains both A and B. The advantage of this over a pure B substratum is that less reference enzyme B is required, resulting in less nonspecific background noise on the solid matrices.

Within the principles of the invention as just described, and as exemplified and as set forth in the preceding discussion, it is possible to use a great many combinations of reference conjugates and reference coupling pairs in connection with various immunochemically binding pairs. It should be understood that the reference coupling pair may bind to each other immunochemically.

The term "reference coupling pair" is used, in connection with this invention, however, to connote a pair of substances which bind to each other selectively, either chemically or immunochemically, which are different from the ligand and receptor pair which are the subject of the assay. Thus, for example, the reference coupling pair may comprise an antigen to which a label, e.g., an enzyme label, has been conjugated, forming the reference conjugate, and an antibody to the antigen immobilized on the reference substratum, or pad. So long as the antigen and antibody are chemically and/or immunochemically different from the ligand to be determined, they may serve as the reference couple.

A particularly attractive group of reference couples are the very large number of haptens and monoclonal antibodies to the haptens. The advantage of using a hapten-anti-hapten as the reference coupling pair is that the coupling between the hapten and the anti-hapten is frequently very specific and not subject to interference or cross reaction with other immunochemical species.

Among the haptens, to which monoclonal antibodies may be grown, which are suitable for use in the present invention, are 2,4-dinitrophenyl, which is exemplified hereinbefore, and a number of fluorescent dyes, e.g., fluorescein, substituted rhodamine, morpholinorhodamine and Texas red.

- 28 -

As indicated in the previous discussion, it is contemplated within the present invention to provide differing quantitative values of reference conjugates. For example, using the materials and techniques described and exemplified before, two reference pads may be provided. One reference pad would contain immobilized antibodies to 2,4-dinitrophenyl (DNP), and the second reference pad would contain immobilized avidin. A first reference conjugate, in a predetermined amount, comprising ALP-DNP conjugate, and a second reference conjugate comprising ALP-biotin conjugate, are added to the test solution. Upon development, the two reference pads will exhibit a detectable level of color of different intensity, each intensity corresponding to a different concentration. By this means, the ligand can be bracketed between or proximate the two reference colors.

### INDUSTRIAL APPLICATION

The present invention finds application in the health care industry and, in particular, in diagnostics. The present invention is suitable as an aid in determining the presence of ligands. Particular application is also found in forensic diagnostics for the detection of drugs of abuse, e.g., morphine, heroin, cocaine, etc. in any fluid, most particularly in body fluids.

CLAIMS

1.     Apparatus for use in detecting ligands in solution comprising:

a solid substratum reference area containing one component of a reference coupling pair of substances which binds specifically to another component of said reference coupling pair; and

a solid substratum test area containing a receptor for the ligand to be detected.

2.     The apparatus of Claim 1 wherein the reference area contains avidin or biotin, adapted to bind, respectively, biotin containing reference conjugate or avidin containing reference conjugate.

3.     The apparatus of Claim 1 wherein the test area receptor comprises monoclonal or polyclonal antibodies to the ligand to be determined.

4.     The apparatus of Claim 3 wherein the test area receptor comprises antibodies to morphine or barbiturate.

5.     A kit for use in detecting a ligand in solution comprising:

a solid substratum reference area containing one component of a reference coupling pair of substances which binds specifically to the other component of the coupling pair, but not a ligand normally found in the solution;

a predetermined quantity of the other component of said reference coupling pair, said other component comprising a reference conjugate containing a detectable label;

a solid substratum test area containing a receptor for the ligand to be detected; and

a predetermined quantity of ligand of the type to be detected labeled with a detectable label.

6. The kit of Claim 5 further comprising at least one additional test area containing a receptor for at least one, different additional ligand to be detected.

7. The kit of Claim 5 wherein the reference area contains avidin or biotin, adapted to bind, respectively, biotin containing reference conjugate or avidin containing reference conjugate.

8. The kit of Claim 5 wherein the test area receptor comprises monoclonal or polyclonal antibodies to the ligand to be determined.

9. A method for detecting ligands in a test solution suspected of containing the test ligand to be detected comprising:

introducing into the test solution a solid substratum reference area containing one component of a reference coupling pair of substances which bind specifically one to another, but which does not bind with a component normally found in the test solution, a predetermined quantity of the other component of said reference coupling pair, said other component comprising a reference conjugate containing a detectable label, a solid substratum test area containing a receptor for the ligand to be detected, and a predetermined quantity of ligand of the type to be detected labeled with a detectable label;

removing the reference area and the test area from the solution; and

detecting the label on the reference area and test area.

10. The method of Claim 9 wherein the reference area contains avidin or biotin, adapted to bind, respectively, biotin containing reference conjugate or avidin containing reference conjugate.

Fig.1

RESPONSE OF INDICATOR PAD
(RABBIT ANTI-MORPHINE)

RESPONSE OF REFERENCE PAD
(AVIDIN)

K/S AT 640nm

(MORPHINE) (ng/mL)

Fig.2

RESPONSE OF INDICATOR PAD
(RABBIT ANTI-MORPHINE)

RESPONSE OF REFERENCE PAD
(MURINE MONOCLONAL Ig G
ANTI-DNP)

K/S AT 640nm

(MORPHINE) (ng/mL)

Fig. 3

Fig. 4

Fig. 5

0203238

European Patent
Office

EUROPEAN SEARCH REPORT

Application number

EP  85 30 7785

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| D,Y | CLINICAL CHEMISTRY, vol. 29, no. 9, September 1983, pages 1598-1603, Washington, US; D.J. LITMAN et al.: "An internally referenced test strip immunoassay for morphine" * complete * | 1-6 | G 01 N  33/52<br>G 01 N  33/543<br>G 01 N  33/94 //<br>G 01 N  33/577 |
| Y | EP-A-0 089 806  (AMERSHAM INTERNATIONAL) * page 4, lines 8-34 * | 1-6 | |
| A | PATENT ABSTRACTS OF JAPAN, vol. 8, no. 105 (P-274)[1542], 17th May 1984; & JP - A - 59 15861 (KONISHIROKU SHASHIN KOGYO K.K.) 26-01-1984 | 7 | |
| P,A | WO-A-8 501 747  (INOMEDIX, INC.) | | **TECHNICAL FIELDS SEARCHED (Int. Cl.4)**<br><br>G 01 N  33/00 |
| A | GB-A-2 018 986  (SYVA CO.)<br><br>& US - A - 4 233 402 (Cat. D) | | |
| P,A | EP-A-0 148 643  (U. LY) * complete * | 1 | |

The present search report has been drawn up for all claims

| Place of search | Date of completion of the search | Examiner |
|---|---|---|
| BERLIN | 15-08-1986 | GREEN C.H. |